# EUROPEAN PATENT APPLICATION

(11) **EP 2 281 465 A1**
(43) Date of publication of application: **09.02.2011**
(21) Application number: 09010104.9
(22) Date of filing: 05.08.2009
(51) Int. Cl.: A23K 1/00, A23K 1/16, A23K 1/18

(54) **Vitamin K3 derivative / NSA formulation**

(71) Applicant: Lonza Ltd., 4052 Basel (CH)
(72) Inventor: The designation of the inventor has not yet been filed

(57) **Abstract**

The present invention discloses a process for the production of formulated vitamin K3 derivatives, whereby nicotinic acid amide (NSA) is used as a formulation agent, as well as NSA-formulated vitamin K3 derivatives obtainable by said process

## Description

The present invention discloses a process for the formulation of vitamin K3 derivatives with nicotinic acid amide (NSA) as well as a product obtainable by said process.

Vitamin K3 (2-methyl-1,4-naphthochinone; menadione) derivatives are used as an ingredient in the animal feed industry. Vitamin K3 derivatives were continuously developed in order to increase the stability of the vitamin. In the 1940s MSB (Menadione Sodium Bisulphite), which possesses a relatively low stability, was introduced in the market. A decade later, MSBC, a bisulphite complex consisting of MSB, NaHSO₃ and water, was offered as a new product form. The stability of this product was slightly higher, but not enough to guarantee a storage time of 4-6 months. In the 1960s and 1970s, MPB (4,6-dimethyl-2-hydroxy-pyrimidine), coated MSB (protective films) and MNB (Menadione-nicotinamide-bisulphite) were developed and produced. Nowadays MNB is the most stable product in the market and exclusively used for example in broiler pallets. However, even during this pelleting process (80°C, high humidity), 50% of MNB is decomposed.

In addition, the vitamin K3 content in a premix is below 1 w%, which causes significant segregation or homogeneity problems in the vitamin premix. Both requirements, low segregation with relatively large particles (100-300 µm) in the range of the other compounds and high homogeneity with very small particles to guarantee a theoretical well distribution, can not be fulfilled at the same time.

Furthermore, small particles lead to lower stability due to a higher specific surface.

According to the state of the art, stability issues are addressed by adding an additional amount of vitamin K3 in order to ensure the minimum K3 concentration. However, this results in additional costs.

In order to determine the stability of the vitamin K3 derivatives, short and long term stability tests are conducted. In short term stability tests, the amount of the vitamin K3 derivatives in a given mixture normally falls below the 10% margin based on the original concentration within 7 to 9 days. In long term tests lasting up to 6 months, loss of up to 40% of the vitamin K3 derivatives is detected, dependent on the mixture.

The US 5,128,151 discloses the use of physiologically tolerated organic or inorganic acids to improve the stability of MSB.

Thus, there exists a need for other processes to enhance the stability of vitamin K3 derivatives that overcome the drawbacks of the state of the art.

Said problem is solved by the process according to the present invention as defined in the claims, which results in a product that is superior to the state of the art in the combination of:
1. higher stability;
2. better segregation properties; due to a formulation process which ensures a narrow particle size distribution resulting in particles with the same size compared to all other particles in the premix, segregation is contained.
3. better availability; even at low vitamin K3 derivative concentrations, a better spreading within the mixtures leads to a uniform distribution of the vitamin.
4. low dust; due to the formulation process, the new product has a low fraction of dust.
5. constant amount of substances in the formulation; due to a formulation consisting only of active substances, no additional filler or coating substance is needed.

Generally, said process is characterized by formulation of the vitamin K3 derivatives with NSA. All formulation techniques known in the state of the art can be employed. However, it may be preferred that the formulation is effected by mixing of water, NSA and the vitamin K3 derivative and removal of the water, subsequent or in stages.

It may be preferred to effect the removal of water in a spray dryer or spray-granulator.

Other preferred methods are high shear granulator or the combination of grinding, kneading, drying and breaking.

It may be preferred that the vitamin K3 derivative/NSA mass ratio is between 2/1 and 1/100, particularly between 1/1 and 1/10.

"Derivative" according to the invention is used in its accepted chemical sense of describing a compound which arises from its parent compound by the replacement of one or more atoms with another atom or group of atoms.

It may be preferred that the vitamin K3 derivative is selected from the group consisting of MNB, MBP, MSBC and MSB.

Another object of the present invention is a NSA-formulated vitamin K3 derivative, which is obtainable by a process according to the invention.

It may be preferred that the formulated vitamin K3 derivative particles have a size of at least 50µm, preferably between 50 and 1000µm and most preferably between 100 and 400µm.

The invention will be further described by the following, non-limiting examples.

### Example 1

### Spraygranulation

- MNB was spray-granulated with a 40 %(w/w) NSA solution (heated to 60°C) at a temperature at 60°C in a laboratory granulator (Aeromatic). After the experiment, the ratio of MSB:NSA was measured to 1:0.87 by HPLC. For further investigations, also the fraction between 100 µm and 315 µm was isolated.
   Combination of grinding, kneading, drying and breaking
- MNB and NSA (ratio 1:2) were grinded in a ball mill (Analysette Kugelmühle), mixed together with water in a kneader (2 h, ∼15% water, 25°C). Afterwards, the product was dried at 50°C and 15 mbar for 16 h in a vacuum drying oven. The product was grinded with a mortar and fractioned in a vibration sieve between 100 µm and 315 µm.

### Accelerated Stability Test

The matrix for the stability tests of niacin-formulated MNB is shown in Table 1.

The composition of the premix is shown below 'premix'.

**Table 1: Test matrix for vitamin K3 stability tests**

| substances | content |
|---|---|
| premix oligo-element | 15.2% |
| choline chloride 50% | 24.3% |
| copper sulfate | 14.2% |
| CaCO3 | 37.4% |
| premix | 8.9% |
| A | 6.46% |
| D3 | 3.09% |
| E 50% | 29.70% |
| B1 | 0.99% |
| B2 | 2.97% |
| Pantothenic acid | 7.42% |
| K3 | 0.00% |
| B6 | 0.99% |
| B12 0.1% | 9.90% |
| niacin | 0.00% |
| folio acid | 0.49% |
| C | 25.61% |
| (ashes) | 12.37% |

For the accelerated stability test, the sample of the mixture above was divided on day 0 in flasks for HPLC-preparation. These flasks were stored in a climate chamber at a constant atmosphere of 25°C and 65% moisture. For each data point, two samples were taken.

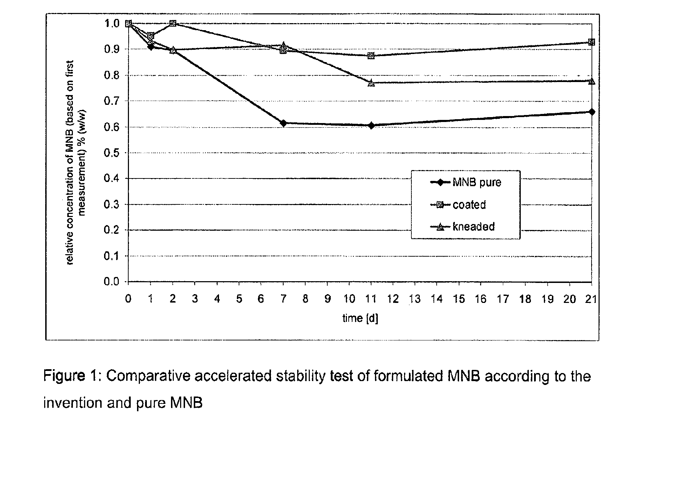

The products were compared in the accelerated stability test with the standard mixture of MNB with NSA and the other substances as presented above. The results are shown in Figure 1.

The experiments indicate a better stability of NSA-pre-formulated MNB than pure MNB. The NSA coating shows better values than just mixing and granulating. After 11 days, the MSB-concentration remains constant in the range of the accuracy of measurement.

## Claims

1. Process for the production of a formulated vitamin K3 derivative, whereby nicotinic acid amide (NSA) is used as a formulation agent.

2. Process according to claim 1, whereby the formulation is effected by mixing of water, NSA and a vitamin K3 derivative and removal of the water, subsequent or in stages.

3. Process according to claim 2, whereby the removal of water after mixing is effected in a spray dryer or spray-granulator.

4. Process according to claim 2, whereby the mixing and formulation is conducted in a high shear granulator and drying is conducted in a convective or contact dryer.

5. Process according to claim 2, whereby the process is a combination of grinding, kneading, drying and breaking.

6. Process according to at least one of claims 1-5, whereby the vitamin K3 derivative/NSA mass ratio is between 2/1 and 1/100, particularly between 1/1 and 1/10.

7. Process according to any of claims 1-6, whereby the vitamin K3 derivative is selected from the group consisting of MNB, MBP, MSBC and MSB.

8. A NSA-formulated vitamin K3 derivative, obtainable by a process according to at least one of claims 1-6.

9. The NSA-formulated vitamin K3 derivative according to claim 8, whereby the formulated vitamin K3 derivative particles have a size of at least 50µm, preferably between 100 and 400µm and most preferably between 200 and 350µm.
